(19)

Europäisches
Patentamt

European
Patent Office

Office européen
des brevets

(11) **EP 4 544 994 A1**

(12) **EUROPEAN PATENT APPLICATION**
published in accordance with Art. 153(4) EPC

(43) Date of publication:
**30.04.2025 Bulletin 2025/18**

(21) Application number: **23851566.2**

(22) Date of filing: **20.07.2023**

(51) International Patent Classification (IPC):
***A61B 5/0215*** (2006.01)

(52) Cooperative Patent Classification (CPC):
**A61B 5/00; A61B 5/0215**

(86) International application number:
**PCT/CN2023/108373**

(87) International publication number:
**WO 2024/032344 (15.02.2024 Gazette 2024/07)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **11.08.2022 CN 202210963169**

(71) Applicant: **Shanghai Microport Cardioadvent Co.,
Ltd.
Shanghai 201203 (CN)**

(72) Inventors:
• **REN, Kaibing**
 **Shanghai 201203 (CN)**
• **ZHU, Zexun**
 **Shanghai 201203 (CN)**
• **LU, Fei**
 **Shanghai 201203 (CN)**
• **YAO, Yao**
 **Shanghai 201203 (CN)**
• **LI, Junfei**
 **Shanghai 201203 (CN)**

(74) Representative: **Marks & Clerk LLP**
 **15 Fetter Lane**
 **London EC4A 1BW (GB)**

(54) **IMPLANTABLE SENSING APPARATUS**

(57)      Provided is an implantable sensing apparatus, which includes a measuring member composed of a package housing and an oscillator disposed in the package housing. The package housing has a probing portion for sensing an external pressure change and experiencing a strain. The oscillator is adapted to convert the strain of the probing portion into a change in its own resonant frequency. The device also includes an anchoring member, which is coupled to the measuring member and disposed outside the package housing. The anchoring and measuring members are adapted to clamp opposite sides of a target septum along its thickness direction. The implantable sensing apparatus has a simpler pressure measurement circuit, which enables more reliable and stable pressure monitoring. Moreover, pressure measurements of the device are not significantly affected by surface endothelialization, resulting in a reduced impact on hemodynamics and a lower risk of thrombosis.

Fig. 1

EP 4 544 994 A1

Description

TECHNICAL FIELD

The present invention relates to the field of medical devices, and particularly to an implantable sensing apparatus for pressure monitoring.

BACKGROUND

Heart failure (HF) is a syndrome of impaired cardiac circulation caused by venous blood congestion and insufficient arterial blood supply as a consequence of the inability of the heart to sufficiently pump the venous return due to systolic and (or) diastolic function dysfunction. HF is a severe manifestation or advanced stage of various heart diseases and has been associated with high mortality and readmission rates. In developed countries, the incidence of heart failure is 1.5-2.0% among overall population, and is as high as ≥10% among those aged ≥70. An epidemiological survey conducted in 2003 showed that HF affected 0.9% of adults at the age of 35 to 74 in China. As population aging accelerates, the incidence of chronic diseases such as coronary heart disease, hypertension, diabetes and obesity has been on the rise in China. Another cause for increasing HF prevalence in China is that the increasingly improved medical care in this country has extended the lifespan of patients with heart disease.

Acute decompensation of heart failure may lead to shortness of breath, edema and fatigue, the most common reasons for hospitalization of HF patients. At present, HF prevention, as well as hospital admission criteria for HF patients, relies mainly on frequent assessments. However, the hospitalization rate of HF patients remains high. During HF progression from stable hemodynamics, before acute decompensation occurs, which may lead to symptoms that require hospitalized treatment, such as edema (fluid retention) or shortness of breath, increases in filling pressure are usually experienced 20-30 days, and then thoracic impedance changes 10-20 days, before the necessary hospitalization. Therefore, monitoring of cardiac pressure (e.g., of the left atrium or pulmonary artery) can provide a time for early medical intervention in HF patients. Appropriate left atrial pressure (LAP) or pulmonary arterial pressure (PAP) control can alleviate blood congestion, reducing the need for hospitalized treatment or the period of required hospitalization. Regarding HF, LAP monitoring is more straightforward and reliable than PAP monitoring. This is because about 90% of patients admitted for heart failure have been found with pulmonary congestion related to increased LAP. Given the increased pulmonary resistance, PAP-based left-sided filling pressure estimation would become unreliable, as has been demonstrated in more than 50% of patients with advanced heart failure. Additionally, straightforward cardiac pressure information allows for more sensitive detection of other pathological entities such as mitral regurgitation, myocardial ischemia and atrial arrhythmia.

There have been passive (i.e., battery-free) LAP monitoring devices, which provide an extended service life and can be reliably implanted. However, such devices still suffer from the problems as follows: (1) they typically incorporate a dedicated integrated circuit for data processing, which tends to be complex and cannot provide reliable and stable pressure measurements; and (2) they typically employ an elongate cylindrical sensor capable of sensing pressure at one end. However, due to a small sensing area, pressure measurements are susceptible to endothelialization. Although the influence of post-implantation endothelialization on the sensing end can be mitigated by bringing the sensing end deeper into the left atrium, this, however, may adversely affect left atrial hemodynamics and lead to an increased risk of thrombosis.

SUMMARY

It is an objective of the present invention to provide an implantable sensing apparatus for pressure monitoring, which overcomes the above-described problems with conventional implantable pressure monitoring device, including a complex circuit with unsatisfactory reliability and stability and pressure measurements that are susceptible to endothelialization.

To this end, the present invention provides an implantable sensing apparatus, which includes:

a measuring member composed of a package housing and an oscillator disposed in the package housing, the package housing including a probing portion for sensing an external pressure change and experiencing a strain, the oscillator adapted to convert the strain of the probing portion into a change in its own resonant frequency; and
an anchoring member coupled to the measuring member, the anchoring member disposed outside the package housing, the anchoring and measuring members adapted to clamp two opposite sides of a target septum along a thickness direction thereof.

In one embodiment, the package housing may be composed of a top plate and a base, wherein part of the top plate provides the probing portion, and the probing portion has a thickness less than a peripheral thickness of the top plate.

In one embodiment, the peripheral thickness of the top plate may be 1.0 $\mu$m to 1.5 $\mu$m greater than the thickness of the probing portion.

In one embodiment, the top plate may have at least one of:

an axial length of 5 mm to 20 mm;
a transverse width of 2 mm to 5 mm; and
a maximum thickness of 0.05 mm to 0.5 mm.

In one embodiment, the top plate may have at least one of:

an axial length of 6.0 mm to 12.0 mm;
a transverse width of 2.0 mm to 4.0 mm; and
a maximum thickness of 0.05 mm to 0.20 mm.

In one embodiment, the top plate may define, at its inner side, an upper recess corresponding to the probing portion, and the base may define, at its inner side, a lower recess in communication with the upper recess, wherein the oscillator is composed of a capacitor and an inductor, which are connected in parallel, the capacitor including a first electrode disposed in the upper recess and a second electrode disposed in the lower recess, and wherein the inductor is disposed between the upper and lower recesses, with its opposite ends being electrically connected to respective terminals of the capacitor.

In one embodiment, one or two second electrodes may be included, wherein:

when one second electrode is included, one end of the inductor is electrically connected to a first terminal of the first electrode, and the other end of the inductor is electrically connected to a second terminal of the second electrode; or
when two second electrodes are included, the two ends of the inductor are electrically connected to respective second terminals of the respective second electrodes.

In one embodiment, an amount of strain of the first electrode may be less than an initial distance between the first and second electrodes, which is 0.05 $\mu$m to 50 $\mu$m.

In one embodiment, the initial distance between the electrodes may be 0.05 $\mu$m to 10 $\mu$m.

In one embodiment, when one second electrode is included, the first electrode may include one first terminal electrically connected to one end of the inductor, and the second electrode may include one second terminal electrically connected to the other end of the inductor, wherein the first and second terminals are arranged on different sides of the first and second electrodes.

In one embodiment, the inductor may be composed of a hollow coil body and taps provided at respective opposite ends of the coil body, the coil body consisting of a wire wound on a support structure and surface-coated with an insulating layer, the support structure disposed in the lower recess, wherein the second electrode is arranged on a top end face of the support structure.

In one embodiment, the coil body may have a line diameter of 0.01 mm to 0.2 mm, and/or the number of turns in the coil body may be 5 to 100.

In one embodiment, the lower recess may have at least one of:

an axial length of 6.0 mm to 10.0 mm;
a depth of 0.5 mm to 2.0 mm; and
a transverse width of 2.0 mm to 4.0 mm.

In one embodiment, the upper recess may have at least one of:

a depth of 1.0 $\mu$m to 1.5 $\mu$m;
a transverse width of 1.5 mm to 2.5 mm; and
an axial length of 3.0 mm to 6.0 mm.

In one embodiment, wiring channels may be formed in solid portions of the top plate along the periphery of the upper recess. The wiring channels may have a greater depth than the upper recess.

In one embodiment, the wiring channels may have a depth of 0.02 mm to 0.08 mm, a transverse width of 0.3 mm to 0.5 mm and an axial length of 1.5 mm to 2.5 mm.

In one embodiment, the top plate may have a Young's modulus of 50 GPa to 90 GPa.

In one embodiment, the top plate may be made of one of glass, quartz, quartz glass, sapphire, monocrystalline silicon, polyetheretherketone (PEEK) and polymethyl methacrylate (PMMA), or a combination thereof. Additionally or alternatively, the base may be made of one of glass, quartz, quartz glass, sapphire, monocrystalline silicon, PEEK and PMMA, or a combination thereof.

In one embodiment, the anchoring member may include a clamping portion and a connecting portion joined to the clamping portion, wherein the package housing is provided with connecting holes in side walls thereof at locations proximal to a bottom wall thereof, which transversely extend through the side walls; the connecting portion is passed through the connecting holes and thereby rotatably connected to the package housing; and the clamping portion and the measuring member are adapted to clamp two opposite sides of a target septum along a thickness direction thereof.

In one embodiment, the measuring member may have a total height of 1.6 mm to 2.7 mm.

In one embodiment, the probing portion may be strained 0.1 nm to 5 nm per mmHg.

In summary, the present invention provides an implantable sensing apparatus, which includes: a measuring member composed of a package housing and an oscillator disposed in the package housing, the package housing including a probing portion for sensing an external pressure change and experiencing a strain, the oscillator adapted to convert the strain of the probing portion into a change in its own resonant frequency; and an anchoring member coupled to the measuring member, the anchoring member disposed outside the package housing, the anchoring and measuring members adapted to clamp a septum of interest on opposite sides thereof along its thickness direction. With this arrangement, the implantable sensing apparatus of the present invention has a simplified circuit structure because it is unnecessary to incorporate a dedicated processing integrated circuit in the device. This ensures post-implantation reliability stability of the implantable sensing apparatus. Moreover, the probing portion is insignificantly affected by endothelialization, and a long distance of protrusion into the left atrium is avoided, resulting in a reduce impact on hemodynamics in the left atrium and a lower risk of thrombosis. Further, more accurate pressure measurements can be provided.

## BRIEF DESCRIPTION OF THE DRAWINGS

The accompanying drawings are provided to facilitate a better understanding of the present invention and not intended to limit the scope thereof in any sense, in which regions that are not visible are indicated by dashed boxes.

Fig. 1 is a schematic overall structural view of an implantable sensing apparatus according to an embodiment of the present invention.

Fig. 2 is a schematic overall structural view of a measuring member according to an embodiment of the present invention.

Fig. 3 is a cross-sectional view of the measuring member according to an embodiment of the present invention, in which a single first electrode and a single second electrode are arranged in one-to-one correspondence.

Fig. 4 is a top view of a top plate according to an embodiment of the present invention.

Fig. 5 is a top view of a base according to an embodiment of the present invention.

Fig. 6 shows dimensions of the top plate according to an embodiment of the present invention.

Fig. 7 shows dimensions of the base according to an embodiment of the present invention, which includes a single second electrode.

Fig. 8 is a cross-sectional view of the measuring member according to an embodiment of the present invention, in which a first electrode and a second electrode are in a one-to-one arrangement, with first and second terminals being arranged on opposite sides of the electrodes.

Fig. 9 is a top view of the base according to an embodiment of the present invention, which includes two second electrodes.

Fig. 10 is a cross-sectional view of the measuring member according to an embodiment of the present invention, in which a first electrode and two second electrodes are in a one-to-two arrangement.

Fig. 11 is a schematic structural view of an inductor according to an embodiment of the present invention.

Fig. 12 is a schematic structural view of the inductor according to an embodiment of the present invention, which is electrically connected to one first electrode and one second electrode.

Fig. 13 is a schematic structural view of the inductor according to an embodiment of the present invention, which is electrically connected to two second electrodes.

Fig. 14 is a schematic structural view of the base according to an embodiment of the present invention.

Fig. 15 is a schematic structural view of a support structure arranged in a lower recess in the base according to an embodiment of the present invention.

Fig. 16 is a schematic structural view of the base and the support structure according to an embodiment of the present invention, which are formed integrally.

Fig. 17 shows dimensions of the integrally formed base and support structure according to an embodiment of the present invention.

Fig. 18 is a schematic structural view of the top plate formed therein with wiring channels according to an embodiment of the present invention, in which regions that are not visible are indicated by dashed boxes.

Fig. 19 is a schematic structural view of an inductor according to an embodiment of the present invention, which is wound on the support structure arranged in the base and electrically connected to two second electrodes.

Fig. 20a shows a scenario in which the implantable sensing apparatus is applicable according to an embodiment of the present invention.

Fig. 20b is an enlarged partial view of the scenario of Fig. 20a.

Fig. 21a shows simulation results at a maximum top plate thickness of 0.13 mm according to an embodiment of the present invention.

Fig. 21b shows simulation results at a maximum top plate thickness of 0.15 mm according to an embodiment of the present invention.

Fig. 22 is an equivalent circuit diagram of an oscillator employing the one-to-two arrangement of first and second electrodes according to an embodiment of the present invention.

List of Reference Numerals

100 implantable sensing apparatus; 200 measuring member; 210 package housing; 211 top plate; 2111 upper recess; 2112 wiring channel; 212 base; 2121 lower recess; 2122 first side wall; 2123 second side wall; 2124 third side wall; 2125 fourth side wall; 2126 bottom wall; 2127 connecting hole; 213 probing portion; 220 oscillator; 221 capacitor; 2211 first electrode; 2211a first terminal; 2212 second electrode; 2212a second terminal; 222 inductor; 2221 coil body; 2222 tap; 2223 support structure; 300 anchoring member; L axial length of top plate; W transverse width of top plate; t maximum thickness of top plate; $d_0$ initial distance between electrodes; Ls axial length of base; Ws transverse width of base; Hs height of base; $L_G$ axial length of lower recess; $W_G$ transverse width of lower recess; $W_C$ transverse width of upper recess; $L_C$ axial length of upper recess; $W_P$ transverse width of wiring channel; $L_P$ axial length of wiring channel.

## DETAILED DESCRIPTION

Objectives, features and advantages of the present invention will become more apparent upon reading the following detailed description of the present invention in conjunction with the accompanying drawings. Note that the figures are provided in a very simplified form not necessarily drawn to exact scale for the only purpose of helping explain the disclosed embodiments in a more convenient and clearer way. As used herein, the singular forms "a", "an" and "the" include plural referents, unless the context clearly dictates otherwise. As used herein, the term "or" is generally employed in the sense including "and/or", unless the context clearly dictates otherwise. As used herein, the term "plurality" means "two or more", and the term "a number of" refers to a non-limited number, unless otherwise specified. The following description sets forth numerous specific details in order to provide a more thorough understanding of the present invention. However, it will be apparent to those skilled in the art that the present invention can be practiced without one or more of these specific details. In other instances, well-known technical features have not been described in order to avoid unnecessary obscuring of the invention.

In the following, for ease of illustration, the terms "axial", "circumferential", "transverse", "proximal" and "distal" may be used. When used to describe an implantable sensing apparatus, "axial" refers to a direction along an axis thereof, which is, when the device is loaded in a sheath, substantially parallel to, or collinear with, an axis of the sheath. "Circumferential" refers to a direction about, and "transverse" refers to a direction perpendicular to, the axis of the implantable sensing apparatus. For example, when the implantable sensing apparatus is loaded in the sheath, "transverse" refers to a direction along which a cross-section of the sheath is taken. "Proximal" refers to an end of the implantable sensing apparatus closer to an operator, while "distal" refers to an end thereof away from the operator, when it is being delivered within the sheath.

In principle, this application seeks to provide an implantable sensing apparatus, which is much simpler in circuit structure by not containing any battery or integrated circuit, thereby providing more reliable and more stable pressure measurements. In particular, the implantable sensing apparatus allows surface endothelialization because such endothelialization has little impact on its pressure measurements. This makes it unnecessary for the implantable sensing apparatus to protrude a considerable distance into a cavity of interest (e.g., the left atrial cavity), effectively reducing the impact that it may otherwise exert on hemodynamics in the cavity and lowering the risk of thrombosis. Moreover, this can result in additional increases in the accuracy of pressure measurements. It should be noted that the implantable sensing apparatus disclosed herein is used for cardiac pressure monitoring including, but not limited to, left atrial pressure (LAP) monitoring.

This application will be further described below with reference to the accompanying drawings, which illustrate preferred embodiments thereof. If there is no conflict, the embodiments described hereunder and features thereof can complement or be combined with each other. Although the following description is set forth in the context of LAP monitoring for the purpose of illustration, those skilled in the art can adapt it to pressure monitoring at different locations in the heart.

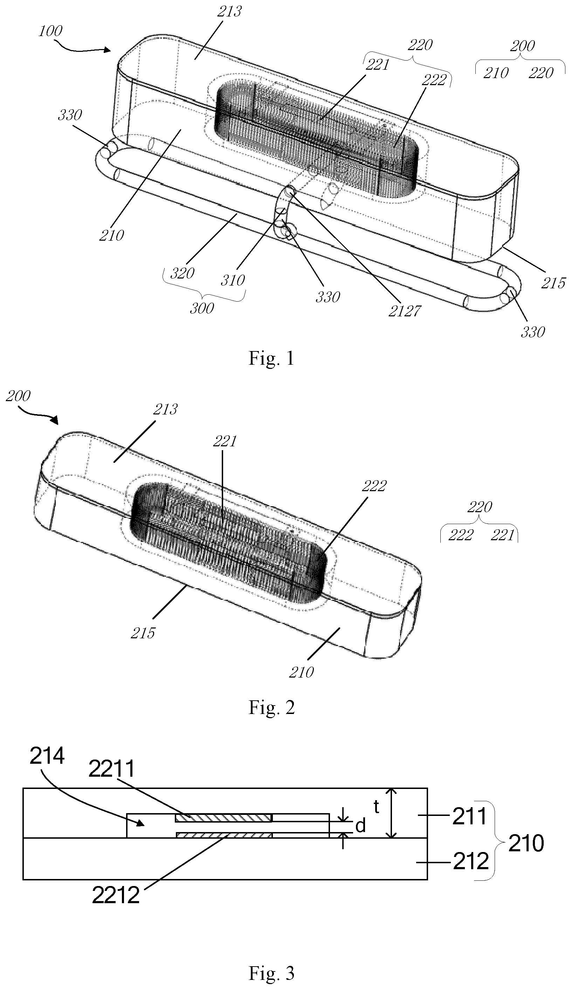

Referring to Figs. 1 and 2, embodiments herein provide an implantable sensing apparatus 100 for LAP monitoring, which includes a measuring member 200 and an anchoring member 300. The measuring member 200 is composed of a package housing 210 and an oscillator 220 and does not contain any integrated circuit or battery. That is, the measuring member 200 contains the oscillator 220 as its only electrical component. The oscillator 220 is disposed within the package housing 210, and the package housing 210 is provided to seal and support the oscillator 220. The oscillator 220 is preferred to be an LC oscillator composed of a capacitor 221 and an inductor 222, the capacitor 221 and the inductor 222 are connected in parallel. The package housing 210 has a probing portion 213 (e.g., a top portion), the probing portion 213 is capable of sensing an LAP change and producing a corresponding strain. The oscillator 220 is used to convert the strain of the probing portion 213 into a change in its own resonant frequency. The oscillator 220 can be measured by an external measuring device and serves as a basis for LAP estimation. This enables wireless LAP monitoring.

In one specific embodiment, the probing portion 213 is connected to the capacitor 221. The capacitor 221 is adapted to convert the strain of the probing portion 213 into a change in its own capacitance, which in turn causes the change in resonant frequency of the oscillator 220. It will be understood that, since the inductor 222 and the capacitor 221 are connected in parallel, the inductor 222 serves both as a resonant element and as an antenna for wireless energy and signal transmission, during measurement. When a measuring device (e.g., another inductor) is brought into proximity of the body of a patient, in which the implantable sensing apparatus 100 is deployed, energy and signals can be transmitted between it and the inductor 222 in the implantable sensing apparatus 100 through near-field coupling. In this way, electrical energy can be supplied to the measuring member 200, and resonant frequency values can be read from the measuring member 200, enabling wireless LAP monitoring.

In addition, the anchoring member 300 is coupled to the measuring member 200 and disposed outside the package housing 210. Preferably, the anchoring member 300 is rotatably coupled to the measuring member 200 in order to facilitate release of the measuring member 200 and then the anchoring member 300 from a sheath. The implantable sensing apparatus 100 can assume either a configuration where it is confined within the sheath, or a configuration where it has been released from the sheath. The measuring member 200 may have its own axis running parallel to an axis of the sheath when in the confined configuration and extending perpendicular to the latter when in the released configuration. In this way, after released from the sheath, the measuring member 200 can spontaneously rotate and adapt itself to orient the probing portion 213 toward the left atrium.

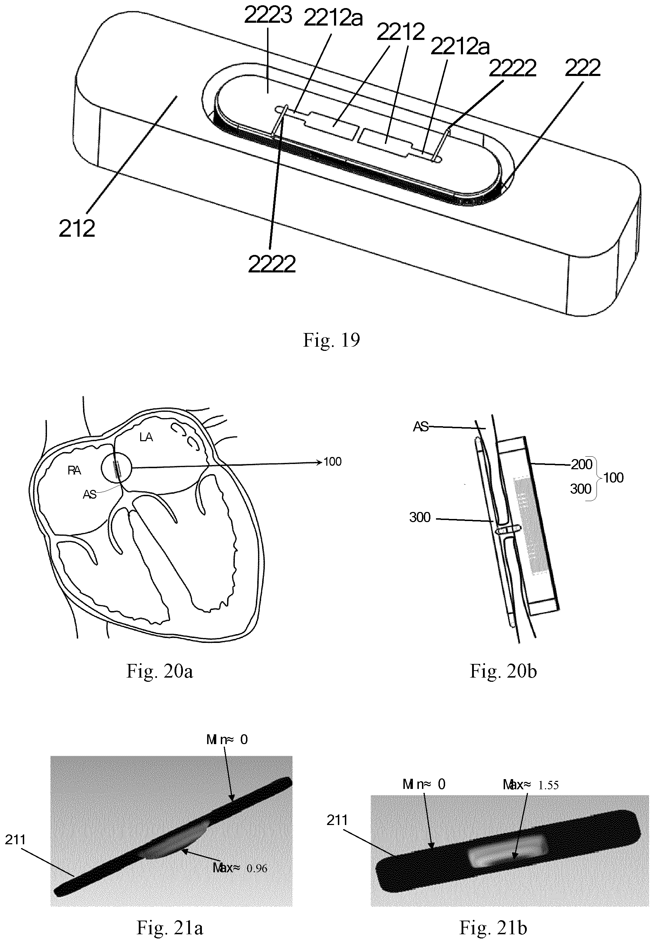

In practical use, as shown in Figs. 20a and 20b, the anchoring member 300 and the measuring member 200 may be deployed on opposite sides of an atrial septum (AS, i.e., a septum of interest) in its thickness direction to clamp the AS therebetween, thereby anchoring the entire implantable sensing apparatus 100 to the AS. The present invention is not limited to any particular structure of the anchoring member 300. For example, the anchoring member 300 may be a braided structure, or a simpler structure such as a linear rod or a sheet. Preferably, it is a linear rod. The anchoring member 300 is elastic so as to be able to be folded and compressed in a sheath and recover the original shape once it is released from the sheath. As can be appreciated, this application enables the measuring member 200 to be used directly as another anchoring member, which clamps tissue of the atrial septum together with the anchoring member 300. With this arrangement, the number of components in the device can be reduced to provide a simpler mechanical structure, which allows the device to less affect the body of a patient after implantation. Moreover, the probing portion 213 is allowed to be made bigger and sufficiently stiff. In this way, even when endothelialization occurs to the surface of the measuring member 200, its pressure measurements will be less affected. This makes it unnecessary for the measuring member 200 to protrude a considerable distance into the left atrium, effectively reducing the impact that it may otherwise exert on hemodynamics therein and lowering the risk of thrombosis. Moreover, this can result in additional increases in the accuracy of pressure measurements.

Differing from conventional elongate cylindrical sensors, as the measuring member 200 additionally serves to provide clamping, the measuring member 200 is provided as a block-like structure with a relatively small height, a relatively large axial length and a relatively large transverse width, instead of an elongate cylindrical structure. The axial length and transverse width of the block-like structure define a bottom portion 215 for clamping the AS, and the probing portion 213 is defined opposite to the bottom portion 215 to provide pressure sensing. Therefore, the measuring member 200 is allowed to have an even smaller height and not protrude a considerable distance into the left atrium along its height.

This application is not limited to any particular shape of the measuring member 200, and the measuring member 200 may be provided as a block-like structure of various shapes. For example, it may have a rectangular, elliptical, circular or another regular shape, or an irregular shape. As would be appreciated, the regular shape is axisymmetric or centrosymmetric, but the irregular shape is not. Therefore, the proposed implantable sensing apparatus 100 has a simpler structure capable of more reliable and more stable pressure monitoring. Moreover, pressure detection is accomplished by a pressure-sensitive surface portion of the measuring member 200, instead of by an end portion thereof. This makes the probing portion 213 less susceptible to endothelialization and ensures accurate pressure measurements. Additionally, it is unnecessary for the implantable sensing apparatus 100 to protrude a considerable distance into the left atrium along its height, effectively reducing the impact that it may otherwise exert on hemodynamics therein and lowering the risk of thrombosis.

Referring to Figs. 3 to 5, the capacitor 221 is composed of a first electrode 2211 and a second electrode 2212. The second electrode 2212 maintains the same pose (including position and orientation) during pressure measurement. The first electrode 2211 is attached to the probing portion 213 so as to be strained as the probing portion 213 deforms. Moreover, the amount of strain depends on pressure being applied. When the first electrode 2211 is strained, a distance between the first electrode 2211 and the second electrode 2212 will vary, causing a change in the capacitance of the capacitor 221. That is, the capacitor 221 is essentially a parallel-plate capacitor with a variable distance between the plates. The capacitance C can be calculated according to:

$$C = \frac{\varepsilon_0 \varepsilon_r S}{d}$$

where $\varepsilon_0$ is the vacuum permittivity that is equal to $8.85 \times 10^{-12}$ F/m, $\varepsilon_r$ is a relative dielectric constant, which is dependent on a material filled between the capacitor electrodes, S is an area of the electrodes, and $d$ is the distance between the electrodes. The capacitance varies with the distance between the electrodes, while the resonant frequency of the measuring member 200, which satisfies the following equations, in turn varies with the capacitance:

$$f = \frac{1}{2\pi\sqrt{LC(p)}} = \frac{K}{\sqrt{C(p)}},$$

and

$$K = \frac{1}{2\pi\sqrt{L}},$$

where L represents the inductance, C denotes the capacitance; $\pi$ is taken to be 3.14, $p$ is the left atrial pressure (LAP), and $C(p)$ describes variation of the capacitance as a function of LAP. There is a correspondence relationship between the capacitance and the pressure, which may have been determined in advance by calibration. During measurement, as the inductance $L$ remains the same, $K$ is a determined constant. A change in the capacitance C can be calculated from a measured change in the resonant frequency $f$, and a LAP change can be in turn determined based on the change in the capacitance C.

In an exemplary embodiment, depending on LAP variation, the inductance L may be configured in the range of 0.1-1000 $\mu$H and the capacitance C in the range of 0.1-1000 pF, and the resonant frequency may be accordingly configured to vary within the range of 0.1-2000 MHz. Alternatively, depending on LAP variation, the inductance L may be configured in the range of 1-10 $\mu$H and the capacitance C in the range of 1-10 pF, and the resonant frequency may be accordingly configured to vary within the range of 16~160 MHz.

This application is not limited to any particular method for measuring the resonant frequency, and those skilled in the art will know how to measure the resonant frequency based on the common general knowledge in the art. For example, the resonant frequency of the implantable sensing apparatus 100 may be measured using any well-known method in the art, such as a time-domain method, frequency sweeping or the like. Further description thereof is deemed unnecessary and omitted herein.

Referring to Fig. 3, the package housing 210 is composed of a top plate 211 and a base 212. Part of the top plate 211 provides the probing portion 213. A thickness of the probing portion 213 is less than a thickness of the top plate 211 along its peripheral edges. In particular, the peripheral thickness of the top plate 211 may be 1.0-1.5 $\mu$m (i.e., 0.001-0.0015 mm) greater than that of the probing portion 213. In one specific embodiment, the top plate 211 defines an upper recess 2111 at its inner side, which corresponds to the probing portion 213. The base 212 defines a lower recess 2121 at its inner side, which is in communication with the upper recess 2111. The upper recess 2111 and the lower recess 2121 enclose a cavity for accommodating the capacitor 221 and the inductor 222. The first electrode 2211 is provided in the upper recess 2111, and the second electrode 2212 is provided in the lower recess 2121, the inductor 222 is inserted between the lower recess 2121 and the upper recess 2111. The inductor 222 is considered as being located within the lower recess 2121, but in some cases, the inductor 222 is also considered as being partially received within the upper recess 2111 and connected to the first electrode 2211. Opposite ends of the inductor 222 are electrically connected to respective terminals of the capacitor 221. It will be understood that the top plate 211 provides an insulating substrate for the first electrode 2211, and vice versa, i.e., an insulating substrate for the first electrode 2211 provides the top plate 211.

Either one or two second electrodes 2212 may be included. When there is a single second electrode 2212, one end of the inductor 222 is electrically connected to a terminal of the first electrode 2211, and the other end of the inductor 222 is electrically connected to a terminal of the second electrode 2212. When there are two second electrodes 2212, the two ends of the inductors 222 are electrically connected to respective terminals of the respective second electrodes 2212. Moreover, both second electrodes 2212 are provided in opposition to the first electrode 2211, forming two capacitors connected in series.

As noted above, the top plate 211 does not have a constant thickness. Instead, it is a trough-like structure thinner around the center and thicker along the periphery. The thinner central portion of the top plate 211 serves as the probing portion 213, the probing portion 213 can be easily strained by pressure due to the smaller thickness. Therefore, the probing portion 213 forms a pressure-sensitive region. The top plate 211 is attached to the base 212 along the thicker peripheral region, which can provide sufficient support, as desired.

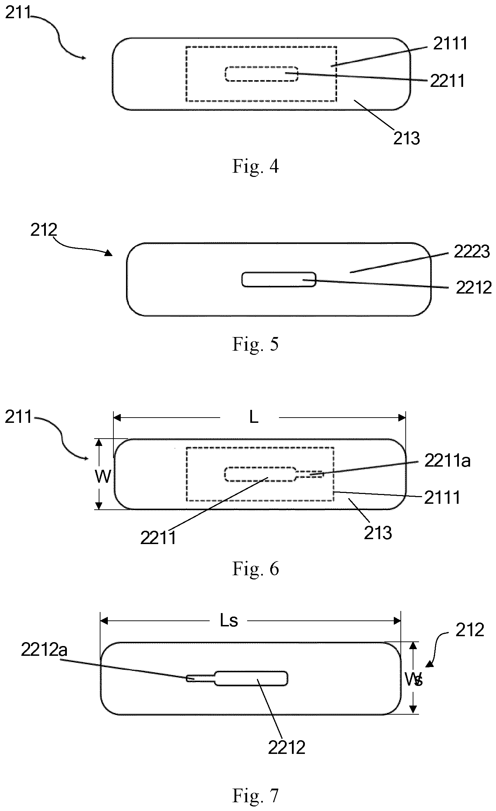

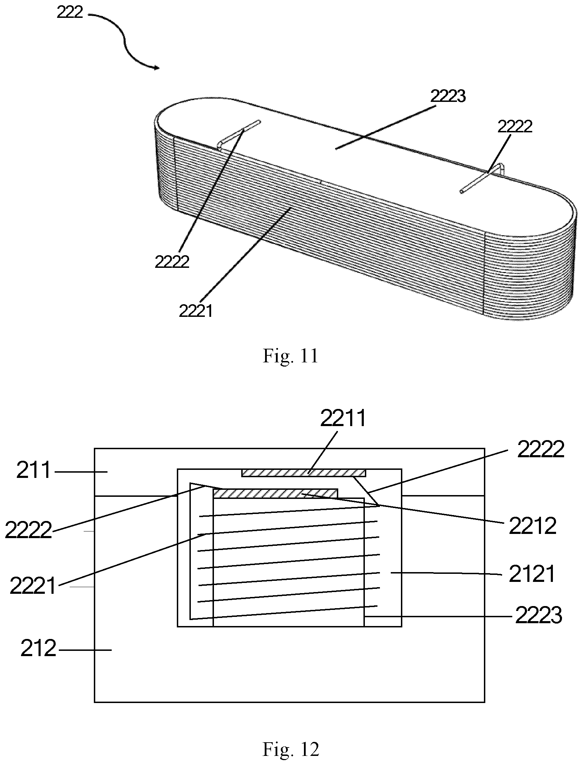

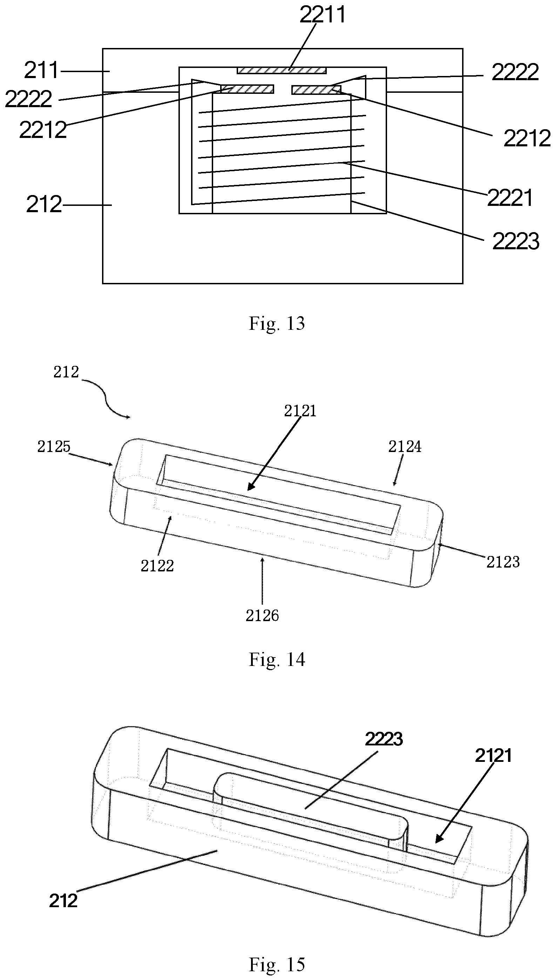

As shown in Figs. 3 to 4, in embodiments of this application, the first electrode 2211 is directly arranged on the bottom of the upper recess 2111. With this arrangement, the first electrode 2211 can be strained at the same time as the probing portion 213 in the upper recess 2111 is strained by pressure. The top plate 211 and the first electrode 2211 may be fabricated either integrally or separately. As shown in Figs. 3 and 5, in embodiments of this application, the second electrode 2212 may be directly arranged on the bottom of the lower recess 2121 so that the base 212 can directly serve as an insulating substrate for the second electrode 2212. However, in alternative embodiments, the second electrode 2212 may be indirectly arranged in the lower recess 2121 through a support structure 2223 (see Figs. 12 and 13), and in these cases, the support structure 2223 instead serves as an insulating substrate for the second electrode 2212.

The substrates or package housing 210 may be made of an insulating material, and the present application is not limited to any particular type of the insulating material. The insulating material is desired to exhibit good mechanical properties, and may be accordingly selected from glass, quartz, quartz glass, sapphire, monocrystalline silicon, polyetheretherketone (PEEK), polymethyl methacrylate (PMMA) and the like, and combinations thereof. This application is also not limited to any particular electrode material, and each electrode may be made of a metal material with good conductivity, such as gold, silver, copper or an alloy material. This application is also not limited to any particular method of attaching the electrodes to the substrates or package housing 210, and those skilled in the art will know how to accomplish this based on the common general knowledge in the art. For example, the attachment of the electrodes to the substrates or package housing 210 may be accomplished with any of many available material deposition techniques, including electroplating, chemical vapor deposition (CVD), physical vapor deposition (PVD), ion sputtering, molten metal deposition, etc.

In order to ensure sufficient sensitivity of the top plate 211, the axial length and/or transverse width of the top plate 211 may be increased to allow the top plate 211 to be more strained at a given pressure thereby augmenting the sensitivity of the measuring member 200 to pressure changes. However, the axial length L and the transverse width W of the top plate 211 are desired not to exceed associated upper limits in order to avoid compromising the ability of the implantable sensing apparatus 100 to be delivered within a sheath and implanted into a left atrium (because excessive dimensions thereof will adversely affect blood flow therein). For these reasons, the axial length L of the top plate 211 may be configured in the range of 5 mm to 20 mm, such as, for example, 5 mm, 6 mm, 10 mm, 12 mm, 15 mm or 20 mm. Additionally, the transverse width W may be configured in the range of 2 mm to 5 mm, such as, for example, 2 mm, 3 mm, 4 mm or 5 mm.

The thickness of the top plate 211 also affects its pressure measurement sensitivity. As would be appreciated, a thinner top plate 211 is more sensitive to a given pressure. However, if the top plate 211 is too thin, the package housing 210 will be vulnerable to intra- or post-operative damage, and the top plate 211 itself may not be stiff enough to provide the advantage of overcoming the susceptibility of measurements to endothelialization. For these reasons, a maximum thickness t of the top plate 211 may be configured in the range of 0.05 mm to 0.5 mm, such as, for example 0.05 mm, 0.1 mm, 0.13 mm, 0.15 mm, 0.2 mm, 0.25 mm, 0.3 mm, 0.35 mm, 0.4 mm, 0.45 mm or 0.5 mm, and the thickness of the probing portion 213 may be 1.0 $\mu$m to 1.5 $\mu$m less than the maximum thickness.

More preferably, in order to reduce the influence of endothelialization on straining of the top plate 211, the top plate 211 may have a Young's modulus of 50 GPa to 90 GPa. Preferably, the probing portion 213 is strained 0.1 nm to 5 nm per mmHg. A larger rate of strain may increase the influence of endothelialization on the top plate 211, while a smaller rate of strain may reduce the sensitivity of the probing portion 213.

In addition to the above, other factors, including the relative dielectric constant $\varepsilon_r$, the electrode area S and the distance d between the electrodes, also have an impact on the pressure measurement sensitivity. As the electrode area S is proportional to the capacitance C, the sensitivity can be augmented by increasing the electrode area S. The distance d between the electrodes is related to the capacitor's sensitivity and pressure measurement range. During measurement, it is desirably ensured that an amount of strain of the first electrode 2211 caused by a possible maximum pressure is less than an initial distance $d_0$ between the first electrode 2211 and the second electrode 2212. Therefore, an increased initial distance $d_0$ between the electrodes would mean both a wider pressure measurement range and decreased sensitivity of the capacitor. Accordingly, in order to avoid compromising the sensitivity, the initial distance $d_0$ between the electrodes should be configured in an appropriate range, preferably 0.05 $\mu$m to 50 $\mu$m, more preferably 0.1 $\mu$m to 10 $\mu$m. Furthermore, the capacitance also varies depending on the dielectric material being used in the capacitor. In consideration of biocompatibility, air, vacuum or the like may be preferably used as the dielectric material in the capacitor.

Referring to Figs. 6 to 8, in one specific embodiment, both the top plate 211 and the substrate for the second electrode 2212 are made of quartz glass. The axial length L of the top plate 211 is 6.0 mm to 12.0 mm, and the base 212 has an axial length Ls, which is equal to the axial length L of the top plate 211. The transverse width W of the top plate 211 is 2.0 mm to 4.0 mm, and the base 212 has a transverse width Ws, which is equal to the transverse width W of the top plate 211. The maximum thickness t of the top plate 211 is 0.05 mm to 0.20 mm, and the base 212 has a height Hs ranging from 0.20 mm to 2.0 mm. Both electrodes are gold electrodes fabricated using PVD, which are rectangular in shape, have an axial length of 3.0 mm to 5.0 mm, a transverse width of 0.5 mm to 2.0 mm and a thickness of 200 nm to 400 nm and initially spaced from each other at a distance $d_0$ of 0.5 $\mu$m to 1.5 $\mu$m.

With continued reference to Figs. 6 to 8, the first electrode 2211 has a single first terminal 2211a, the single first terminal 2211a is electrically connected to one end of the inductor 222, and the second electrode 2212 has a single second terminal 2212a, the single second terminal 2212a is electrically connected to the other end of the inductor 222. The first terminal 2211a and the second terminal 2212a may be arranged on the same or opposite sides of first electrode 2211 and the second electrode 2212. Preferably, they are arranged on opposite sides, in order to avoid inadvertent contact, which may cause a short circuit.

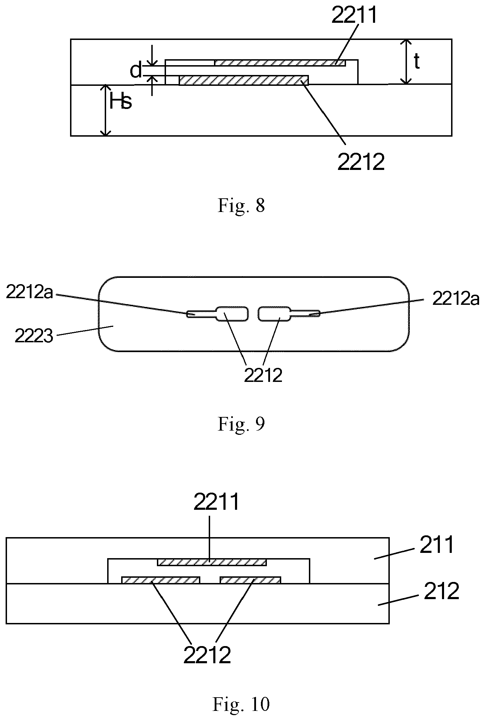

In some embodiments of the present application, a single first electrode 2211 and a second electrode 2212 are provided in a "one-to-one" arrangement. One end of the inductor 222 is electrically connected to the first terminal 2211a of the first electrode 2211 and the other end thereof is connected to the second terminal 2212a of the second electrode 2212, thus forming an LC oscillator 220 composed of a single capacitor 221 and the inductor 222, which are connected in parallel. Referring to Figs. 9 and 10, in conjunction with Fig. 4, in another embodiment of this application, a single first electrode 2211 and two second electrodes 2212 are provided in a "one-to-two" arrangement, in which the two second electrodes 2212 commonly share the one first electrode 2211. The two ends of the inductor 222 are connected to respective second terminals 2212a of the respective second electrodes 2212, thereby forming an LC oscillator 220 composed of two series-connected capacitors 221 and the inductor 222 that is connected in parallel to the capacitors 221. This arrangement enables easier subsequent assembly because connections are established between the inductor 222 and only the second electrodes 2212 in the capacitors 221. In practice, the inductor 222 may have taps connected to the respective terminals in the capacitor(s) 221. Optionally, the electrical connections between the inductor 222 and the capacitor(s) 221 may be established by soldering, conductive adhesive bonding, laser welding, etc., and the inductor 222 and the capacitor(s) 221 can be connected by one or more methods. Preferably, the inductor 222 is connected to the capacitor(s) 221 by laser welding.

Fig. 11 shows an exemplary structure of the inductor 222 according to an embodiment of the present application. As shown in Fig. 11, the inductor 222 is composed of a hollow coil body 2221 and two taps 2222, each end of the coil body 2221 is provided with one tap 2222. The coil body 2221 may consist of a wound wire surface-coated with an insulating layer. The present invention is not limited to any method for winding the wire. For example, the winding may be either helical or not. In order for increased measurement accuracy to be achieved, it is desirable to minimize any other factors that may cause resonant frequency changes than capacitance. This requires the inductor 222 to maintain a stable shape. However, since the wire of the inductor 222 is relatively soft, a support structure 2223 may be provided in the hollow cavity of the coil body 2221. That is, the wire may be wound on the support structure 2223 into the coil body 2221. The support structure 2223 can function to fix the coil body 2221 and maintain the geometric stability of the coil body 2221. The support structure 2223 may have a shape matching that of the hollow cavity of the coil body 2221. Referring to Fig. 12, the support structure 2223 may be disposed in the lower recess 2121, and the second electrode 2212 may be provided on a top end face of the support structure 2223 either directly or indirectly. In this way, the support structure 2223 can directly or indirectly serve as an insulating substrate for the second electrode 2212.

According to embodiments of the present application, the second electrode 2212 may be directly arranged on the support structure 2223, and the support structure 2223 and the base 212 may be formed either integrally or separately.

It will be understood that dimensions and electrical properties of the inductor 222 are affected by many factors including the material and diameter of the wound wire, a thickness of the insulating layer, a line diameter (defined as the sum of the diameter of the wire and the thickness of the insulating layer), an encircled area (defined as an area of the hollow cavity delimited by the wire) and the number of turns. Optionally, the wire of the coil body 2221 may be made of gold, silver, copper or an alloy. Optionally, the line diameter of the coil body 2221 may range from 0.01 mm to 0.2 mm. Optionally, in order to maximize the encircled area, the coil body 2221 may be formed, for example, by helically winding the wire forth and back (e.g., in a similar way to the formation of a paperclip). Optionally, the number of (winding) turns in the coil body 2221 may range from 5 to 100.

The support structure 2223 is made of an insulating material, which may be same as or different from that of the package housing 210. Preferably, the support structure 2223 and the package housing 210 are made of the same material, which is preferred to be quartz glass. The coil body 2221 may be reinforced by being connected to the support structure 2223, for example, by adhesive bonding, fusion bonding or the like. Alternatively, it may not be reinforced at all. That is, the coil body 2221 may be obtained by directly winding the wire on the support structure 2223.

Referring to Fig. 12, for example, in the one-to-one arrangement, the second electrode 2212 is arranged on the support structure 2223, the support structure 2223 is attached to the base 212. The two taps 2222 of the inductor 222 are respectively connected to the first terminal 2211a and the second terminal 2212a, and the coil body 2221 is wound on the support structure 2223. The support structure 2223 and the base 212 may be separately fabricated and then secured together, for example, by adhesive bonding, fusion bonding or welding. Alternatively, the support structure 2223 and the base 212 may be integrally formed. Preferably, in order for structural simplicity and enhanced reliability to be achieved, the integral formation of the support structure 2223 and the base 212 may be accomplished, for example, using machining, laser processing, chemical etching, injection molding or 3D printing.

Referring to Fig. 13, for example, in the one-to-two arrangement, the second electrodes 2212 are arranged on the support structure 2223, the support structure 2223 is attached to the base 212. The two taps 2222 of the inductor 222 are attached to the respective second terminals 2212a of the respective second electrodes 2212. The support structure 2223 and the base 212 may be formed either integrally or separately.

Referring to Figs. 14 to 16, the base 212 has five walls: four side walls and a bottom wall. Optionally, N ($\leq$5) of the walls of the base 212 may each be separately formed, or integrally formed with another wall. Preferably, the five walls of the base 212 are integrally formed. Optionally, the base 212 may be fabricated separately from or integrally with the support structure 2223. In the former case, the support structure 2223 may be attached to an inner surface of the bottom wall of the base 212, for example, by adhesive bonding, fusion bonding or welding.

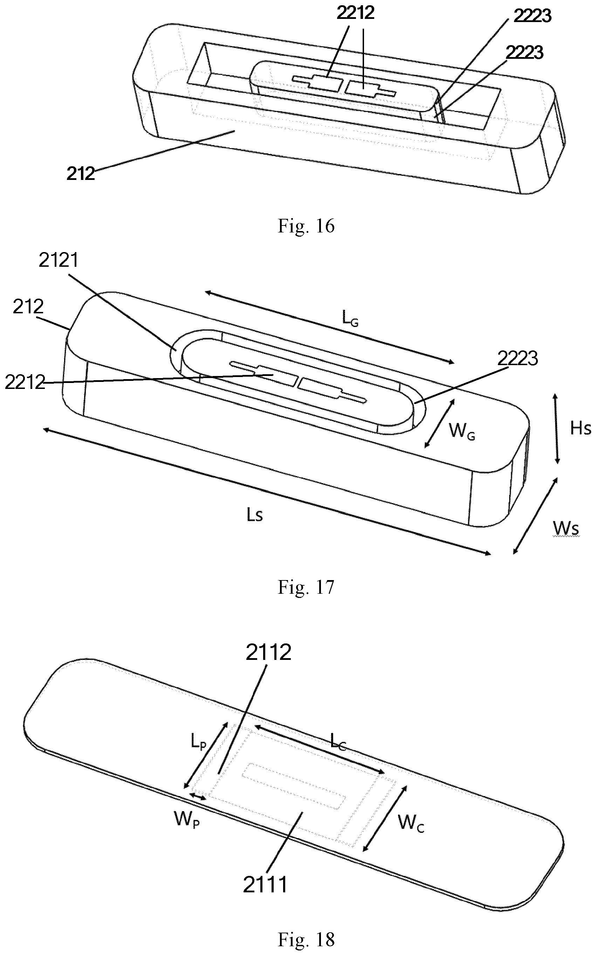

In greater detail, as shown in Fig. 14, the base 212 may have a first side wall 2122, a second side wall 2123, a third side wall 2124 and a fourth side wall 2125, which are sequentially joined to one another, and a bottom wall 2126 joined to all the side walls. The bottom wall 2126 is intended to be brought into contact with tissue of an atrial septum (AS). The second side wall 2123 and the fourth side wall 2125 may oppose each other in a lengthwise direction, and the first side wall 2122 and the third side wall 2124 may oppose each other in a widthwise direction. As shown in Figs. 15 and 16, the support structure 2223 is disposed in the lower recess 2121, the support structure 2223 is integrally formed with the base 212.

Fabrication and assembly of the implantable sensing apparatus 100 are described below with reference to Figs. 17 to 20.

As shown in Fig. 17, the base 212 may be made of quartz glass, and the support structure 2223, the base 212 and the second electrodes 2212 are integrally formed. Specifically, a CVD process may be first carried out on a thick (e.g., 1.5-2.5 mm) quartz glass plate to form the second electrodes 2212, and the "one-to-two" arrangement is used. The second electrodes 2212 may be rectangular, the second electrodes 2212 each have an axial length of 1.5 mm to 3.0 mm, a transverse width of 0.5 mm to 2.0 mm and a thickness of 200 nm to 400 nm. Additionally, each second electrode 2212 may have a second terminal 2212a. The quartz glass plate is processed by laser processing, chemical etching, cutting, etc. into a desired shape and to form the lower recess 2121 for accommodating the inductor 222. Optionally, the axial length Ls of the base 212 is 12.0 mm to 18.0 mm, the transverse width Ws of the base 212 is 3.0 mm to 5.0 mm and the height Hs thereof is 1.0 mm to 3.0 mm, and the axial length $L_G$ of the lower recess 2121 is 6.0 mm to 10.0 mm, the depth (not labeled) of the lower recess 2121 is 0.5 mm to 2.0 mm and the transverse width $W_G$ of the lower recess 2121 is 2.0 mm to 4.0 mm.

As shown in Fig. 18, the top plate 211 may be made of quartz glass, and the top plate 211 and the first electrode 2211 may be integrally formed. Specifically, the upper recess 2111 may be formed in a thin (e.g., 0.10-0.20 mm) quartz glass plate by dry etching, wet etching, laser engraving, grinding, etc. Preferably, the upper recess 2111 has a depth (not labeled) of 1.0 $\mu$m to 1.5 $\mu$m, a transverse width $W_C$ of 1.5 mm to 2.5 mm and an axial length $L_C$ of 3.0 mm to 6.0 mm. The same or a different technique may be then used to form wiring channels 2112 in solid portions of the top plate 211 along the periphery of the upper recess 2111, the wiring channels 2112 may have a greater depth than the upper recess 2111. The wiring channels 2112 may be formed to accommodate connection of the inductor 222 to the capacitor 221. Preferably, the wiring channels 2112 may have a depth (not labeled) of 0.02 mm to 0.08 mm, a transverse width $W_P$ of 0.3 mm to 0.5 mm and an axial length $L_P$ of 1.5 mm to 2.5 mm. After the upper recess 2111 and the wiring channels 2112 are formed, a PVD process may be carried out to form the first electrode 2211 on an inner surface of the upper recess 2111. The first electrode 2211 may have the same transverse width as the second electrodes 2212 and an axial length which is twice of the axial length of the second electrodes 2212 or longer. Subsequently, the top plate 211 may be processed into a desired shape, for example, using laser processing, chemical etching or cutting.

As shown in Fig. 19, the inductor 222 is placed into the lower recess 2121 in the integrally-formed quartz glass base 212, and the support structure 2223 is tightly secured in the lower recess 2121. The two taps 2222 of the inductor 222 may be then connected to the respective second terminals 2212a of the respective second electrodes 2212 using laser welding.

Referring back to Fig. 1, after the above steps are completed, the top plate 211 is placed on the base 212, the top plate 211 is in flush alignment with the base 212. The top plate 211 and the base 212 are then sealingly connected to each other, for example, by laser welding, fusion bonding or adhesive bonding. Preferably, the top plate 211 and the base 212 are connected by using laser welding.

With continued reference to Fig. 1, connecting holes 2127 may be provided in side walls of the base 212 at locations close to the bottom wall 2125, which extend through the walls transversely. Additionally, the anchoring member 300 may include a connecting portion 310 and a clamping portion 320, which are joined to each other. The connecting portion 310 may be passed through the connecting holes 2127 and thereby rotatably connected to the base 212. Optionally, the anchoring member 300 may essentially consist of an elastic wire. In a non-limiting example, the elastic wire is made of a nickel-titanium alloy. In order to enable visualization in an implantation procedure, the anchoring member 300 is preferably provided with a radiopaque structure 330 on at least one of the connecting portion 310 and the clamping portion 320. The radiopaque structure 330 may be made of a radiopaque metal material. The present invention is not limited to any particular radiopaque metal material, and those with good biocompatibility are preferred, such as gold, platinum, iridium and alloys thereof. In a preferred embodiment of the present application, radiopaque structures 330 are provided respectively at proximal and distal ends of the clamping portion 320, and another radiopaque structure 330 is provided on the connecting portion 310. The present invention is not limited to any particular shape of the radiopaque structures 330, and they may be provided, for example, in the form of radiopaque sleeves or bands.

Figs. 20a and 20b schematically illustrate a post-implantation pose in the heart according to an embodiment of the present application. In these figures, RA denotes the right atrium; LA, the left atrium; and AS, the atrial septum. As shown in Figs. 20a and 20b, the implantable sensing apparatus 100 is implanted into the heart so as to be anchored to the atrial septum (AS), the probing portion 213 of the measuring member 200 is oriented towards the left atrium. The measuring member 200 may have a total height of 1.6 mm to 2.7 mm, which will not significantly affect hemodynamics in the left atrium. Additionally, endothelialization is allowed a certain period of time after the implantation. Therefore, the implantable sensing apparatus 100 of this application is allowed to have a significantly reduced post-implantation height.

Detailed experimental data is set forth below to demonstrate advantages of the implantable sensing apparatus 100 described herein.

1. Mechanical properties

(1) Resistance to Influence of Endothelialization

It will be understood that, when endothelial tissue grows across the surface of the measuring member 200, pressure measurement deviations before and after the endothelialization principally depend on the stiffness difference between the endothelial tissue on the surface of the top plate and the top plate 211 itself. If the top plate 211 is much stiffer than the endothelial tissue, then the influence of the endothelialization on straining of the top plate 211 will be almost negligible. As pressure-induced capacitance changes just result from changes in the distance d between the electrodes caused by straining of the top plate 211, in this case, the capacitance will vary as a function of pressure substantially in the same way even after the endothelialization takes place. That is, it can be considered that pressure measurements will not be affected by the endothelialization.

For example, when the top plate 211 is rectangular, the cross-sectional shape of the top plate 211 will remain rectangular after it is endothelialized. Its bending stiffness is proportional to its Young's modulus E and to the cube of its thickness t ($t^3$). Therefore, the bending stiffness EI is proportional to $E*t^3$. The Young's moduli of various materials suitable for the top plate 211 in embodiments of this application and of myocardial tissue are presented in Table 1 below.

Table 1: Young's moduli of Top Plate and Myocardial Tissue

| Material | Glass | Quartz | Quartz Glass | Sapphire | Mono-Si | PMMA | PEEK | Myocardial Tissue |
|---|---|---|---|---|---|---|---|---|
| Young's modulus E (GPa) | 50-70 | 66-75 | 72 | 350 | 160 | 2.9 | 3.6 | $4\times10^{-5}$ |

As can be seen from Table 1, the suitable insulating materials for the top plate 211 all exhibit a much higher Young's modulus E than myocardial tissue. That is, when the top plate 211 is made of any of the materials, the influence of endothelialization on its straining will be almost negligible. However, at a given thickness, the bending stiffness of the top plate 211 is affected by the Young's modulus. For these reasons, the Young's modulus of the top plate 211 is preferred to be in the range of 50 GPa to 90 GPa. Accordingly, the material of the top plate 211 may be selected from glass, quartz and quartz glass.

In preferred embodiments of the present application, the maximum thickness t of the top plate 211 is 0.05 mm to 0.20 mm. Typically, the endothelial tissue has a thickness less than 1.0 mm. Accordingly, $E*t^3$ values of the materials for the top plate 211 and the endothelial layer (assumed to have a thickness of 1.0 mm) are calculated as shown in Table 2.

Table 2 E*t³ Values of Various Materials for Top Plate and Myocardial Tissue

| Material | Glass | Quartz | Quartz Glass | Sapphire | Mono-Si | PMMA | PEEK | Myocardial Tissue |
|---|---|---|---|---|---|---|---|---|
| E*t³ (GPa • mm³) | 0.05 - 0.56 | 0.066 - 0.6 | 0.072 - 0.576 | 0.35 - 2.8 | 0.18 - 1.44 | 0.0029 - 0.0232 | 0.0036 - 0.0288 | 0.00004 |

Bending stiffness (E*t³) ratios of the endothelial layer to the materials for the top plate 211 are summarized in Table 3.

Table 3 Bending Stiffness Ratios of Endothelial Layer to Various Materials for Top Plate

| Material | Glass | Quartz | Quartz Glass | Sapphire | Mono-Si | PMMA | PEEK |
|---|---|---|---|---|---|---|---|
| Ratio ($\times 10^{-4}$) | 0.7 - 8 | 0.67 - 6.1 | 0.7 - 5.6 | 0.14 - 1.1 | 0.27 - 2.2 | 17 - 138 | 14 - 111 |

According to the data of Table 3, monocrystalline silicon has the smallest bending stiffness ratio (0.27), and PMMA has the greatest ratio (138). Considering that the top plate 211 will be hardly strained by pressure if it is too stiff, it is preferred to be made of glass, quartz or quartz glass. When any of these materials is used, the bending stiffness of the top plate will vary by less than 0.08% under the effect of the endothelial layer. Therefore, this influence is almost negligible. In consideration of various factors, including biocompatibility, safety and manufacturing cost, the top plate is preferred to be made of quartz glass.

(2) Thickness and Amount of Strain of Top Plate

As noted above, the thickness of the top plate 211 will directly affect its amount of strain when it is stressed. When the thickness of the top plate 211 is relatively small, it will be more strained at a given pressure, i.e., more pressure-sensitive. However, if the thickness is too small, the resistance to the influence of endothelialization may be degraded, and the top plate 211 may become vulnerable to damage during and after implantation.

Figs. 21a and 21b shows amounts of strain of the top plate per mmHg at some thicknesses within a preferred thickness range (0.05-0.20 mm) according to embodiments of the present application. For example, when the maximum thickness t of the top plate 211 is 0.15 mm, a maximum amount of strain Max of the probing portion 213 per mmHg is 0.96 nm. When the maximum thickness t of the top plate 211 is 0.13 mm, a maximum amount of strain Max of the probing portion 213 per mmHg is 1.55 nm. Therefore, a smaller thickness t of the top plate 211 will lead to the probing portion 213 experiencing a larger amount of strain. Additionally, the non-sensitive region is substantially not strained at all. That is, a minimum amount of strain Min of the top plate 211 is 0 nm.

2. Electrical Properties

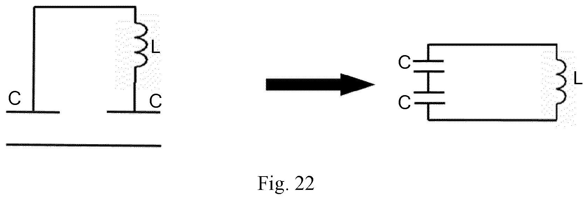

Taking the one-to-two arrangement as an example, as shown in Fig. 22, which shows a simplified circuit diagram of the arrangement, the inductor 222 is connected to the two second terminals 2212a of the second electrodes 2212, forming an LC oscillator 220. The two second electrodes 2212 forms capacitors with the first electrode 2211, which have the same capacitance C and are connected in series. As well known in the electrical art, the total capacitance of two series-connected capacitors of the same capacitance C is equal to half the capacitance of each individual capacitor.

Let the initial distance between the second electrodes 2212 and the first electrode 2211 be $d_0$, then the initial total capacitance $c_0$ of the series-connected capacitors can be expressed as:

$$C_0 = \frac{\varepsilon_0 \varepsilon_r S}{2d_0},$$

where $\varepsilon_0$ is the vacuum permittivity, $\varepsilon_r$ is the relative dielectric constant, and $S$ is the area of the base electrode. By substituting this equation into the equation for calculating the resonant frequency of an LC oscillator, we can obtain the initial resonant frequency $f_0$ of the LC oscillator as:

$$f_0 = \frac{1}{2\pi\sqrt{LC}} = \frac{1}{2\pi\sqrt{L}} \frac{1}{\sqrt{C_0}} = \frac{1}{2\pi\sqrt{L}} \sqrt{\frac{2d_0}{\varepsilon_0 \varepsilon_r S}}.$$

Assuming the distance between the electrodes becomes $d_1$ as a result of the first electrode 2211 being stressed, the total capacitance $C_1$ of the series-connected capacitors will change to:

$$C_1 = \frac{\varepsilon_0 \varepsilon_r S}{2d_1}.$$

Accordingly, the resonant frequency $f_1$ of the LC oscillator becomes:

$$f_1 = \frac{1}{2\pi\sqrt{LC}} = \frac{1}{2\pi\sqrt{L}}\frac{1}{\sqrt{C_1}} = \frac{1}{2\pi\sqrt{L}}\sqrt{\frac{2d_1}{\varepsilon_0 \varepsilon_r S}}.$$

Therefore, a resonant frequency change $\Delta f$ before and after the stressing of the LC oscillator can be obtained as:

$$\Delta f = \frac{1}{2\pi\sqrt{L}}\left(\frac{1}{\sqrt{C_0'}} - \frac{1}{\sqrt{C_1'}}\right) = \frac{1}{2\pi}\sqrt{\frac{2}{L\varepsilon_0\varepsilon_r S}}\left(\sqrt{d_0} - \sqrt{d_0 - \Delta d}\right),$$

where $\Delta d = d_0 - d_1$ represents an amount of strain of the first electrode 2211. An external measuring device can determine pressure changes from such resonant frequency changes. At a given pressure change, a greater resonant frequency change $\Delta f$ means higher pressure measurement sensitivity of the sensing apparatus. Further, the measurability of a resonant frequency change $\Delta f$ for the external measuring device also depends on a ratio of the resonant frequency change $\Delta f$ to the initial resonant frequency $f_0$, also called the resonant frequency slope $\partial f$, i.e.,

$$\partial f = \frac{\sqrt{d_0} - \sqrt{d_1}}{\sqrt{d_0}} = 1 - \sqrt{1 - \frac{\Delta d}{d_0}}.$$

From the equations of $f_0$, $\Delta f$ and $\partial f$, it can be seen that, at given values of the other parameters, the initial distance $d_0$ will affect all the three. Specifically, as $d_0$ increases, $f_0$ increases and both $\Delta f$ and $\partial f$ decrease. Therefore, an increased initial distance $d_0$ means reduced pressure sensitivity of the sensing apparatus and degraded ability of the external measuring device to measure resonant frequency changes.

Table 4 presents some initial distances $d_0$ and corresponding initial resonant frequencies $f_0$, resonant frequency changes $\Delta f$ and resonant frequency slopes $\partial f$, wherein the inductance $L = 6.0\ uH$, the base electrode area $S = 6.75 \times 10^{-7}$ m$^2$, the relative dielectric constant $\varepsilon_r = 1.00053$, the vacuum permittivity is a constant equal to $8.85 \times 10^{-12}$ F/m, and the amount of strain $\Delta d = 1.0 \times 10^{-9} m$ per mmHg.

Table 4: Values of $d_0$ and Corresponding Values of $f_0$, $\Delta f$ and $\partial f$

| $d_0(um)$ | 0.05 | 0.1 | 1.0 | 5.0 | 10.0 | 50.0 |
|---|---|---|---|---|---|---|
| $f_0(MHz)$ | 8.4 | 11.9 | 37.6 | 84.1 | 118.9 | 265.9 |
| $\Delta f(KHz)$ | 84.5 | 59.6 | 18.8 | 8.7 | 5.9 | 2.7 |
| $\partial f(\times 10^{-4})$ | 100 | 50 | 5 | 1 | 0.5 | 0.1 |

According to Table 4, when the initial distance $d_0$ is in the range of 0.05 $\mu$m to 10 $\mu$m, $\partial f$ is greater than 0.005%, a value generally believed to allow a measuring device to easily measure resonant frequency changes. In further consideration of the manufacturing process being used, $d_0$ can be preferably configured in the range of 0.1 $\mu$m to 10 $\mu$m.

Therefore, the implantable sensing apparatus disclosed herein has effectively simplified circuit and mechanical structures, can be implanted in a more stable and more reliable way, and allows for a reduced distance of protrusion into the left atrium, which reduces the impact that it may otherwise exert on hemodynamics and lowers the risk of thrombosis. Further, it can provide more accurate pressure measurements, which are almost not affected by surface endothelialization.

It is noted that many modifications and additions may be made by those of ordinary skill in the art without departing from the teachings disclosed hereinabove, and such modifications and additions are intended to be also included within the scope of the present invention. Any and all equivalent changes such as alternations, modifications and variations made in light of the above disclosure by those familiar with the art without departing from the spirit and scope of the invention are all considered as equivalent embodiments of the present invention. Accordingly, any and all equivalent changes such as alternations, modifications and variations made to the disclosed embodiments in accordance with the essential principles of the present invention fall within the scope of the invention.

**Claims**

1. An implantable sensing apparatus, comprising:

   a measuring member composed of a package housing and an oscillator disposed in the package housing, the package housing comprising a probing portion, the probing portion configured for sensing an external pressure change and experiencing a strain, the oscillator configured to convert the strain of the probing portion into a change in its own resonant frequency; and
   an anchoring member connected to the measuring member, the anchoring member disposed outside the package housing, the anchoring member and the measuring member configured to clamp opposite sides of a target septum along a thickness direction thereof.

2. The implantable sensing apparatus according to claim 1, wherein the package housing is composed of a top plate and a base, wherein part of the top plate provides the probing portion, and the probing portion has a thickness smaller than a peripheral thickness of the top plate.

3. The implantable sensing apparatus according to claim 2, wherein the peripheral thickness of the top plate is 1.0 $\mu$m to 1.5 $\mu$m greater than the thickness of the probing portion.

4. The implantable sensing apparatus according to claim 2 or 3, wherein the top plate has at least one of:

   an axial length of 5 mm to 20 mm;
   a transverse width of 2 mm to 5 mm; and
   a maximum thickness of 0.05 mm to 0.5 mm.

5. The implantable sensing apparatus according to claim 4, wherein the top plate has at least one of:

   an axial length of 6.0 mm to 12.0 mm;
   a transverse width of 2.0 mm to 4.0 mm; and
   a maximum thickness of 0.05 mm to 0.20 mm.

6. The implantable sensing apparatus according to claim 2 or 3, wherein an inner side of the top plate defines an upper recess corresponding to the probing portion, and an inner side of the base defines a lower recess in communication with the upper recess,
   wherein the oscillator is composed of a capacitor and an inductor, which are connected in parallel, the capacitor comprising a first electrode disposed in the upper recess and a second electrode disposed in the lower recess, and wherein the inductor is disposed between the upper recess and the lower recess, opposite ends of the inductor being electrically connected to respective terminals of the capacitor.

7. The implantable sensing apparatus according to claim 6, wherein one or two second electrodes are included, wherein:

   when one second electrode is included, one end of the inductor is electrically connected to a first terminal of the first electrode, and the other end of the inductor is electrically connected to a second terminal of the second electrode; or
   when two second electrodes are included, the two ends of the inductor are electrically connected to respective second terminals of the respective second electrodes.

8. The implantable sensing apparatus according to claim 6, wherein an amount of strain of the first electrode is less than an initial distance between the first electrode and the second electrode, and wherein the initial distance is 0.05 $\mu$m to 50 $\mu$m.

9. The implantable sensing apparatus according to claim 8, wherein the initial distance is 0.05 $\mu$m to 10 $\mu$m.

10. The implantable sensing apparatus according to claim 7, wherein when one second electrode is included, the first electrode comprises one first terminal electrically connected to one end of the inductor, and the second electrode comprises one second terminal electrically connected to the other end of the inductor, wherein the first terminal and the second terminal are arranged on different sides of the first electrode and the second electrode.

11. The implantable sensing apparatus according to claim 6, wherein the inductor is composed of a hollow coil body and taps provided at respective opposite ends of the coil body, the coil body consisting of a wire whose surface is coated with an insulating layer, the wire wound on a support structure, the support structure disposed in the lower recess, and wherein the second electrode is arranged on a top end face of the support structure.

12. The implantable sensing apparatus according to claim 11, wherein the coil body has a line diameter of 0.01 mm to 0.2 mm, and/or wherein the number of turns in the coil body is 5 to 100.

13. The implantable sensing apparatus according to claim 6, wherein the lower recess has at least one of:

      an axial length of 6.0 mm to 10.0 mm;
      a depth of 0.5 mm to 2.0 mm; and
      a transverse width of 2.0 mm to 4.0 mm.

14. The implantable sensing apparatus according to claim 6, wherein the upper recess has at least one of:

      a depth of 1.0 $\mu$m to 1.5 $\mu$m;
      a transverse width of 1.5 mm to 2.5 mm; and
      an axial length of 3.0 mm to 6.0 mm.

15. The implantable sensing apparatus according to claim 6, wherein wiring channels are formed in solid portions of the top plate along a periphery of the upper recess, wherein the wiring channels have a greater depth than the upper recess.

16. The implantable sensing apparatus according to claim 15, wherein the wiring channels have a depth of 0.02 mm to 0.08 mm, a transverse width of 0.3 mm to 0.5 mm and an axial length of 1.5 mm to 2.5 mm.

17. The implantable sensing apparatus according to claim 2 or 3, wherein the top plate has a Young's modulus of 50 GPa to 90 GPa.

18. The implantable sensing apparatus according to claim 2 or 3, wherein the top plate is made of one of glass, quartz, quartz glass, sapphire, monocrystalline silicon, polyetheretherketone and polymethyl methacrylate, or a combination thereof, and/or wherein the base is made of one of glass, quartz, quartz glass, sapphire, monocrystalline silicon, polyetheretherketone and polymethyl methacrylate, or a combination thereof.

19. The implantable sensing apparatus according to any one of claims 1 to 3, wherein the anchoring member comprises a clamping portion and a connecting portion, the connecting portion being connected to the clamping portion, side walls of the package housing being provided with connecting holes at locations proximal to a bottom wall thereof, which transversely extend through the side walls; the connecting portion being passed through the connecting holes and thereby rotatably connected to the package housing; and the clamping portion and the measuring member are configured to clamp opposite sides of the target septum along the thickness direction thereof.

20. The implantable sensing apparatus according to any one of claims 1 to 3, wherein the measuring member has a total height of 1.6 mm to 2.7 mm.

21. The implantable sensing apparatus according to any one of claims 1 to 3, wherein the probing portion is strained 0.1 nm to 5 nm per mmHg.

Fig. 1

Fig. 2

Fig. 3

211

2111

2211

213

Fig. 4

212

2223

2212

Fig. 5

211

L

W

2211a

2111

2211

213

Fig. 6

Ls

2212a

212

Ws

2212

Fig. 7

Fig. 8

Fig. 9

Fig. 10

222

2223

2222

2222

2221

Fig. 11

2211

211

2222

2222

2221

212

2212

2121

2223

Fig. 12

Fig. 13

Fig. 14

Fig. 15

Fig. 16

Fig. 17

Fig. 18

Fig. 19

Fig. 20a

Fig. 20b

Fig. 21a

Fig. 21b

Fig. 22

## INTERNATIONAL SEARCH REPORT

| International application No. |
|---|
| **PCT/CN2023/108373** |

**A. CLASSIFICATION OF SUBJECT MATTER**

A61B5/0215(2006.01)i

According to International Patent Classification (IPC) or to both national classification and IPC

**B. FIELDS SEARCHED**

Minimum documentation searched (classification system followed by classification symbols)

IPC: A61B

Documentation searched other than minimum documentation to the extent that such documents are included in the fields searched

Electronic data base consulted during the international search (name of data base and, where practicable, search terms used)

CNTXT, WPABSC, ENTXTC, CJFD: 植入, 压力, 传感器, 封装, 壳体, 基板, 振荡器, 固定, 夹持, 无源, implant, pressure, sensor, transducer, oscillator, packaging, substrate, fix+, clamp+, passive

**C. DOCUMENTS CONSIDERED TO BE RELEVANT**

| Category* | Citation of document, with indication, where appropriate, of the relevant passages | Relevant to claim No. |
|---|---|---|
| PX | CN 219147561 U (MICROPORT SINICA CO., LTD.) 09 June 2023 (2023-06-09) claims 1-21 | 1-21 |
| PX | CN 219021207 U (MICROPORT SINICA CO., LTD.) 16 May 2023 (2023-05-16) description, paragraphs [0003]-[0129], and claims 1-17, and figures 1-28 | 1-21 |
| Y | US 2005187482 A1 (O'BRIEN, David et al.) 25 August 2005 (2005-08-25) description, paragraphs [0014]-[0094], and claims 1-25, and figures 1-28 | 1-21 |
| Y | US 2007118039 A1 (VITAL SENSORS INC.) 24 May 2007 (2007-05-24) description, paragraphs [0133]-[0135], and figures 24-26 | 1-21 |
| A | CN 213430080 U (FUWAI HOSPITAL, CAMS & PUMC et al.) 15 June 2021 (2021-06-15) entire document | 1-21 |
| A | US 2014100627 A1 (PACESETTER, INC.) 10 April 2014 (2014-04-10) entire document | 1-21 |

☐ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **25 October 2023** | **30 October 2023** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

# EP 4 544 994 A1

## INTERNATIONAL SEARCH REPORT
### Information on patent family members

International application No.

**PCT/CN2023/108373**

| Patent document cited in search report | | | Publication date (day/month/year) | Patent family member(s) | | | Publication date (day/month/year) |
|---|---|---|---|---|---|---|---|
| CN | 219147561 | U | 09 June 2023 | None | | | |
| CN | 219021207 | U | 16 May 2023 | None | | | |
| US | 2005187482 | A1 | 25 August 2005 | AU | 2009201750 | A1 | 28 May 2009 |
| | | | | AU | 2004274005 | A1 | 31 March 2005 |
| | | | | CA | 2539261 | A1 | 31 March 2005 |
| | | | | CA | 2539261 | C | 17 May 2011 |
| | | | | AU | 2009201749 | A1 | 28 May 2009 |
| | | | | US | 2009030291 | A1 | 29 January 2009 |
| | | | | US | 9265428 | B2 | 23 February 2016 |
| | | | | WO | 2005027998 | A2 | 31 March 2005 |
| | | | | US | 2006235310 | A1 | 19 October 2006 |
| | | | | US | 7574792 | B2 | 18 August 2009 |
| | | | | EP | 1677852 | A2 | 12 July 2006 |
| | | | | US | 2012016228 | A1 | 19 January 2012 |
| US | 2007118039 | A1 | 24 May 2007 | EP | 1968433 | A2 | 17 September 2008 |
| | | | | EP | 1968433 | B1 | 29 October 2014 |
| | | | | WO | 2007062299 | A2 | 31 May 2007 |
| | | | | AU | 2006318295 | A1 | 31 May 2007 |
| | | | | JP | 2009517137 | A | 30 April 2009 |
| | | | | US | 2011201949 | A1 | 18 August 2011 |
| | | | | US | 8382677 | B2 | 26 February 2013 |
| | | | | US | 7682313 | B2 | 23 March 2010 |
| | | | | US | 2010174201 | A1 | 08 July 2010 |
| | | | | US | 7931597 | B2 | 26 April 2011 |
| | | | | US | 2014081158 | A1 | 20 March 2014 |
| | | | | CA | 2631057 | A1 | 31 May 2007 |
| | | | | CA | 2631057 | C | 29 November 2016 |
| CN | 213430080 | U | 15 June 2021 | None | | | |
| US | 2014100627 | A1 | 10 April 2014 | None | | | |

Form PCT/ISA/210 (patent family annex) (July 2022)